# FASCICULE DE BREVET EUROPEEN

(11) **EP 2 830 575 B1**
(45) Date de publication et mention de la délivrance du brevet: **28.11.2018**
(21) Numéro de dépôt: 13722476.2
(22) Date de dépôt: 29.03.2013
(51) Int. Cl.: A61K 8/34, A61K 8/37, A61K 8/60, A61Q 19/00, A61Q 19/10

(54) **COMPOSITION AUTO-EMULSIONNANTE COMPRENANT UN ESTER DE POLYGLYCEROL ET UN ESTER DE SACCHAROSE**
SELBSTEMULGIERENDE ZUSAMMENSETZUNG MIT EINEM POLYGLYCEROLESTER UND EINEM SACCHAROSEESTER
SELF-EMULSIFYING COMPOSITION COMPRISING AN ESTER OF POLYGLYCEROL AND AN ESTER OF SACCHAROSE

(30) Priorité: 30.03.2012 FR 1200955
(43) Date de publication de la demande: 04.02.2015
(73) Titulaire: Stearinerie Dubois Fils, 36300 Ciron (FR)
(72) Inventeur: MERLAUD, Fabien, 36300 Le Blanc (FR); DUPUIS, Patrick, F-36800 Oulches (FR); LOUBAT-BOULEUC, Nathalie, F-92380 Garches (FR)
(74) Mandataire: Loyer & Abello
(86) Numéro de dépôt international: PCT/FR2013/000083
(87) Numéro de publication internationale: WO 2013/144462

(56) Documents cités:
- EP-A1- 0 541 830
- "Personal care compositions providing high UVA/UVB light protection and radical scavenging effect", IP.COM JOURNAL, IP.COM INC., WEST HENRIETTA, NY, US, 5 mai 2009 (2009-05-05), XP013131429, ISSN: 1533-0001
- MARTINE SEU SALERNO ET AL: "Use of small particle size crosslinked copolymer of acrylic acid and sodium acrylate in skin care and make-up : Covacryl MV series", RESEARCH DISCLOSURE, MASON PUBLICATIONS, HAMPSHIRE, GB, vol. 505, no. 19, 1 mai 2006 (2006-05-01), XP007136159, ISSN: 0374-4353

## Description

La présente invention est relative à une composition auto-émulsionnante d'origine végétale qui trouve des applications notamment dans le domaine de la cosmétique.

Une composition est dite auto-émulsionnante si elle se suffit à elle-même. Elle permet ainsi la stabilité d'une émulsion c'est-à-dire qu'à un certain taux d'incorporation en milieu aqueux cette émulsion est stable sans ajout d'un co émulsionnant ou d'une cire quelconque.

En effet, pour stabiliser une émulsion il est actuellement nécessaire d'incorporer plusieurs produits en plus de l'eau.

Le document EP-541.830-A est relatif à une composition pour réaliser une émulsion eau-dans-huile dont la stabilisation est obtenue par adjonction d'un ester de sorbitane et des esters de PEG.

Aussi un des buts de la présente invention est-il de fournir une composition qui mise en présence d'eau forme spontanément une émulsion stable.

Un autre but de la présente l'invention est de fournir une telle composition exclusivement végétale permettant une mise en oeuvre simple pour un formulateur en fournissant le moins possible d'énergie mécanique et en intégrant tous les ingrédients en monopote (dénomination anglaise «.one pot ») c'est-à-dire dans une même cuve.

Ces buts, ainsi que d'autres qui apparaîtront par la suite sont atteints par une composition auto-émulsionnante comprenant exclusivement, par rapport au poids total des ingrédients :
- 5 à 45 % en poids d'un ester de polyglycérols,
- 5 à 60 % en poids d'un ou de plusieurs esters de saccharose de HLB différente,
- 20 à 50 % en poids d'un ester de glycérol,
- 1 à 30 % en poids d'un alcool gras,
- 1 à 30 % en poids d'un agent tensioactif, laquelle composition est, selon la présente invention, caractérisée par la fait que l'agent tensio-actif comprend un agent choisi dans le groupe constitué par des savons d'acide gras, des savons estérifiés liés par une liaison sulfate et des sels d'alcool gras,
et que tous ces ingrédients sont d'origine végétale.

Avantageusement, la quantité d'ester de polyglycérols est comprise entre 5 et 35% en poids et même entre 10 et 30% en poids.

De préférence, l'ester de polyglycérols est choisi parmi les esters issus de la réaction de polyglycérols comprenant de 2 à 12 unités de glycérol, de préférence de 3 à 6 unités de glycérol, avec au moins une huile végétale, partiellement hydrogénée ou non hydrogénée, d'indice d'iode compris entre 1 et 145, et en particulier entre 5 et 105.

Avantageusement, la quantité d'esters de saccharose est comprise entre 10 et 50% en poids et même entre 20 et 45% en poids.

De préférence, le ou les esters de saccharose sont choisis parmi les esters issus de la réaction du saccharose avec des acides gras comprenant de 10 à 22 atomes de carbone, de préférence de 12 à 20 atomes de carbone, en particulier de 12 à 18 atomes de carbone.

De préférence, les acides gras sont choisis parmi l'acide laurique, l'acide myristique, l'acide palmitique, et l'acide stéarique ou un mélange d'au moins deux de ces acides gras.

Avantageusement, l'ester de glycérol est choisi parmi les esters issus de la réaction de la glycérine avec des acides gras ou de la transestérification de triglycérides d'acides gras et de glycérine, les acides gras comprenant de 8 à 22 atomes de carbone, de préférence de 10 à 20 atomes de carbone, mieux de 14 à 18 atomes de carbone. En particulier de tels acides gras comprennent l'acide myristique, l'acide palmitique, l'acide stéarique ou un mélange d'au moins deux de ces acides.

De préférence, la quantité d'ester de glycérol est comprise entre 15 à 45% en poids, encore mieux de 20 à 40% en poids par rapport au poids total de la composition.

Selon une variante de réalisation, l'ester de glycérol et d'acide gras végétal est estérifié.

Avantageusement, la quantité d'agent tensio-actif est comprise entre 1 et 20% en poids.

De préférence, l'agent tensio-actif est un savon d'acides gras ou savons estérifiés comprenant de 2 à 22 atomes de carbone, et préférentiellement de 3 à 20 atomes de carbone.

Avantageusement, la quantité d'alcool gras est comprise entre 1 à 20% en poids, encore mieux entre 8 et 20% en poids par rapport au poids total de la composition.

De préférence, l'alcool gras comprend de 8 à 22 atomes de carbone, les chaînes carbonées étant saturées ou insaturées ou fonctionnalisées ou linéaires ou un mélange de celles-ci, de préférence de 10 à 18 atomes de carbone, et en particulier de 12 à 18 atomes de carbone.

Une composition selon la présente invention peut être utilisée pour la fabrication de produits cosmétiques, tels que par exemple crème solaire, lait, spray, sérum, baume et crème à usage cosmétique, produit moussant, produits de maquillage et d'hygiène corporelle.

Selon la présente invention l'ester de polyglycérols peut être obtenu à partir d'acide gras ou par trans-estérification à partir d'huile ou de mélanges d'huiles.

Les acides gras utilisés comprennent de 8 à 22 atomes de carbone, de préférence de 10 à 18 atomes de carbone, et en particulier de 12 à 18 atomes de carbone.

Ces acides peuvent être linéaires ou ramifiés, saturés ou insaturés, posséder une ou des fonctions hydroxyles latérales.

Les huiles peuvent être saturées ou insaturées, de liquide à solide à température ambiante, et posséder éventuellement des fonctions hydroxyle, de préférence d'indice d'iode compris entre 1 et 145, et en particulier de 5 à 105.

L'ester est choisi de préférence parmi les esters issu de la réaction de polyglycérols comprenant de 2 à 12 unités de glycérol, de préférence de 3 à 6 unités glycérol avec au moins une huile végétale partiellement hydrogénée ou non hydrogénée d'indice d'iode compris entre 1 et 145, et en particulier de 5 à 105.

Dans la suite de la présente description, chaque corps chimique sera identifié selon son nom INCI et/ou sa dénomination commerciale ou référence.

On peut citer par exemple l'ester issu de la réaction de polyglycérol-3 et d'acide isostéarique (nom INCI : polyglyceryl-3 diisostearate) tel que celui commercialisé par la société Stéarinerie DUBOIS sous la référence DUB ISO G3, l'ester issu du polyglycérol-3 et de l'acide ricinoléïque (nom INCI: polyglyceryl-3 polyricinoleate) tel que celui commercialisé par la société Stéarinerie DUBOIS sous la référence DUB PGPR, l'ester issu de la réaction de trans-estérification entre une huile végétale et un polyglycérol tel que celui commercialisé par la société Stéarinerie DUBOIS sous la référence DUB COCOROSE+ (Palme), ainsi que les esters suivants commercialisé par la société Stéarinerie sous la référence DUBOIS DUB APRILOSE+ (Abricot) ou DUB LILIROSE + (Tournesol).

La quantité d'ester(s) de polyglycérols dans la composition peut aller par exemple de 5 à 45 % en poids, de préférence de 5 à 30% en poids, encore mieux de 10 à 30% en poids par rapport au poids total de la composition.

Selon la présente invention, l'ester de saccharose et d'acide gras végétal est de préférence choisi parmi les esters issus de la réaction de saccharose et d'acide(s) gras comprenant de 10 à 22 atomes de carbone, de préférence de 12 à 20 atomes de carbone, mieux de 12 à 18 atomes de carbone tels que l'acide laurique et /ou l'acide myristique et/ou l'acide palmitique et/ou l'acide stéarique ou un mélange.

Les esters de saccharose et d'acides gras peuvent être choisis parmi les mono-, di-, tri- et tétra-esters, les polyesters et leurs mélanges. On utilise de préférence des esters à faible degré d'estérification comme par exemple des mono-esters, di-esters, tri-esters de saccharose et d'acide gras ou un mélange.

Dans le cas où l'on utilise un ester de saccharose et d'acide gras, on préfère un ester à faible degré d'estérification, en particulier où les mono-esters ou di-esters sont majoritaires.

De préférence, l'ester de saccharose et d'acide gras utilisé dans la présente invention, ou le mélange d'esters de saccharose et d'acides gras de différentes HLB, présente une HLB comprise entre 1 et 16 et de préférence entre 5 et 11.

Comme cela est bien connu, on entend par HLB (Hydrophilic/Lipophilic Balance) des valeurs qui décrivent le caractère hydrophile ou hydrophobe des molécules ou des mélanges, telles que décrites par Griffin (Griffin W.C. : « Classification of Surface-Active Agents by HLB », Journal of the Society of Cosmetic Chemists 1 (1949) : 311-326).

On peut citer à titre d'exemples d'ester ou de mélanges d'esters de saccharose et d'acide gras :
- Le Surfhope SE COSME C-1216, présentant une HLB de 16, dont le nom INCI est sucrose laurate, comprenant environ 80% de monoester, le reste du mélange étant composé de di- et triesters, et commercialisé sous cette dénomination par la société Mitsubichi-Kagaku Foods Corporation,
- Le Surfhope SE COSME C-1416, présentant une HLB de 16, dont le nom INCI est sucrose myristate, comprenant environ 80% de monoester, le reste du mélange étant composé de di- et triesters, et commercialisé sous cette dénomination par la société Mitsubichi-Kagaku Foods Corporation,
- Le Surfhope SE COSME C-2203, présentant une HLB de 3, dont le nom INCI est sucrose behenate, comprenant environ 20% de monoester, le reste du mélange étant composé de di-, triesters et polyesters, et commercialisé sous cette dénomination par la société Mitsubichi-Kagaku Foods Corporation,
- Le Surfhope SE COSME C-1715, présentant une HLB de 15, dont le nom INCI est sucrose oleate, comprenant environ 70% de monoester, le reste du mélange étant composé de di- et triesters, et commercialisé sous cette dénomination par la société Mitsubichi-Kagaku Foods Corporation,
- Le DUB SE 15P, présentant une HLB de 15, dont le nom INCI est sucrose palmitate, comprenant environ 70% de monoester, le reste du mélange étant composé de di- et triesters, et commercialisé sous cette dénomination par la société Stéarinerie DUBOIS,
- Le DUB SE 15S, présentant une HLB de 15, dont le nom INCI est sucrose stearate, comprenant environ 70% de monoester, le reste du mélange étant composé de di- et triesters, et commercialisé sous cette dénomination par la société Stéarinerie DUBOIS,
- Le DUB SE 11S, présentant une HLB de 11, dont le nom INCI est sucrose stearate, comprenant environ 60% de monoester, le reste du mélange étant composé de di- et triesters, et commercialisé sous cette dénomination par la société Stéarinerie DUBOIS,
- Le DUB SE 5S, présentant une HLB de 5, dont le nom INCI est sucrose distearate, comprenant environ 30% de monoester, le reste du mélange étant composé de di-, triesters et polyesters, et commercialisé sous cette dénomination par la société Stéarinerie DUBOIS,
- Le DUB SE 3S, présentant une HLB de 3, dont le nom INCI est sucrose tristearate, comprenant environ 20% de monoester, le reste du mélange étant composé de di-, triesters et polyesters, et commercialisé sous cette dénomination par la société Stéarinerie DUBOIS,

La quantité d'ester(s) de saccharose peut aller par exemple de 5 à 60 % en poids, de préférence de 10 à 50% en poids, encore mieux de 20 à 45% en poids par rapport au poids total de la composition.

Selon un mode de réalisation, l'ester de glycérol et d'acide gras est choisi parmi les esters, que ceux-ci soient totaux ou partiels, issus de la réaction de la glycérine et d'acides gras ou de la transestérification de triglycérides d'acides gras par de la glycérine. Ces acides gras comprennent de 8 à 22 atomes de carbone, de préférence 10 à 20 atomes de carbone, mieux de 14 à 18 atomes de carbone tels que l'acide myristique et/ou l'acide palmitique et/ou l'acide stéarique ou un mélange.

Ces acides peuvent être linéaires ou ramifiés, saturés ou insaturés posséder une ou des fonctions hydroxyles latérales.

Les esters de glycérol peuvent être estérifiés en plus par de l'acide acétique, lactique, citrique ou tartrique. On utilise de préférence l'acide lactique ou l'acide citrique.

On peut citer à titre d'exemples le DUB GMS (INCI : glyceryl stearate), produit commercialisé sous cette dénomination par la société Stéarinerie Dubois, le DUB OG (INCI : glyceryl oleate), produit commercialisé sous cette dénomination par la société Stéarinerie Dubois, le DUB ISG (INCI : glyceryl isostearate), produit commercialisé sous cette dénomination par la société Stéarinerie Dubois, le Radiamuls citrem 2931 (INCI : glyceryl stearate citrate), produit commercialisé sous cette dénomination par la société Oleon, l'Axol C62 pellets (INCI : glyceryl stearate citrate), produit commercialisé sous cette dénomination par la société Evonik France SAS ou le Radiamuls Lactem 2950 (INCI: glyceryl stearate lactate), produit commercialisé sous cette dénomination par la société Oleon.

La quantité d'ester(s) de glycérol estérifié peut aller par exemple de 1 à 50 % en poids, de préférence de 15 à 45% en poids, encore mieux de 20 à 40% en poids par rapport au poids total de la composition. l'agent tensioactif comprend un savon d'acide gras, ou des savons estérifiés, ou des sels d'alcool gras liés par une liaison sulfate.

Ces savons d'acide gras sont choisis parmi les savons issus de la réaction entre la soude ou la potasse et des acides ou diacides, avec éventuellement une ou plusieurs fonctions hydroxyle.

Les acides peuvent comprendre de 2 à 22 atomes de carbone, mieux de 3 à 20 atomes de carbone.

Ces savons peuvent être sous forme de poudre ou en solution.

On peut citer à titre d'exemples :
- l'Amisoft HS 11, produit commercialisé sous cette dénomination par la société Ajinomoto Co (Nom INCI : Sodium stearoyl glutamate),
- l'Amisoft CS 11, produit commercialisé sous cette dénomination par la société Ajinomoto Co (Nom INCI : Sodium cocoyl glutamate),
- l'Amisoft LS 11, produit commercialisé sous cette dénomination par la société Ajinomoto Co (Nom INCI : Sodium Lauroyl glutamate),
- le Radiamuls SL 2990, produit commercialisé sous cette dénomination par la société Oleon (Nom INCI : Sodium stearoyl lactylate)
- la LANETTE E (nom INCI : sodium cetearyl sulfate), produit commercialisé sous cette dénomination par la société BASF,
- le DUB RNA 40, produit commercialisé sous cette dénomination par la société Stéarinerie DUBOIS (nom INCI : Sodium ricinoleate),
- le DUB SNA VE, produit commercialisé sous cette dénomination par la société Stéarinerie DUBOIS (nom INCI : Sodium stearate (and) Sodium palmitate),

La quantité de savon peut aller par exemple de 1 à 30 % mieux de 1 à 20%.

Les alcools gras utilisés comprennent de 8 à 22 atomes de carbone saturés ou insaturés ou fonctionnalisés ou linéaires ou en mélange, de produit commercialisé sous cette dénomination par 10 à 18 atomes de carbone, et en particulier de 12 à 18 atomes de carbone.

On peut citer à titre d'exemple :
- l'alcool Laurique (LOROL C12-99, produit commercialisé sous cette dénomination par la société BASF; NACOL 1299-100, référence de la société SASOL)
- l'alcool cétylique (LOROL C1698, produit commercialisé sous cette dénomination par la société BASF ou VEGAROL 1698, produit commercialisé sous cette dénomination par la société Berg & Schmidt ou ECOROL 1698, produit commercialisé sous cette dénomination par la société ECOGREEN)
- l'alcool stéarylique (LOROL C1898, produit commercialisé sous cette dénomination par la société BASF ou VEGAROL 1898, produit commercialisé sous cette dénomination par la société Berg & Schmidt ou KALCOL 8098, produit commercialisé sous cette dénomination par la société KAO CHEMICALS)
- l'alcool cétostéarylique. (LOROL C1618C5, produit commercialisé sous cette dénomination par la société BASF ou VEGAROL 161, référence de la société Berg & Schmidt)
- l'alcool oléique (HD-OCENOL 90/95/V, produit commercialisé sous cette dénomination par la société BASF ou REFANOL 90/95/V, produit commercialisé sous cette dénomination par la société ECOGREEN).

La quantité d'alcool gras peut aller par exemple de 1 à 30 % en poids, de préférence de 1 à 20% en poids, encore mieux de 8 à 20% en poids par rapport au poids total de la composition.

Les exemples ci-après qui n'ont qu'un rôle illustratif et n'ont aucun caractère limitatif, ont pour but de permettre à l'homme du métier de mieux comprendre l'invention et les avantages de celle-ci.

Dans un récipient adapté, on charge l'ester de polyglycérol, l'ester de glycérol et l'alcool gras. L'ensemble des ingrédients est mis à fondre puis sous agitation rapide, on introduit l'ester de saccharose. Lorsque le mélange est homogène, on introduit l'agent tensioactif et on refroidit.

Dans le tableau I ci-après sont regroupés différents exemples de formulation de compositions selon la présente invention :

**Tableau I**

| | Exemple 1 | Exemple 2 | Exemple 3 | Exemple 4 | Exemple 5 | Exemple 6 | Exemple 7 | Exemple 8 |
|---|---|---|---|---|---|---|---|---|
| Polyricinoléate de polyglycérol-3 | 20 % | | | | | | | |
| Diisostéarate de polyglycérol-3 | | 12 % | | | | | | |
| Huile de palme polyglycérique | | | 23 % | | | 20 % | 18 % | 20 % |
| Huile d'abricot polyglycérique | | | | 20 % | 20 % | | | |
| Stéarate de glycérol | 20 % | | 32 % | 20 % | | 32 % | 23 % | |
| Stéarate de glycérol estérifié | | 36 % | | | 32 % | | | 32 % |
| Alcool cétylique | 10 % | 15 % | 14 % | 10 % | 14 % | 14 % | 18 % | |
| Alcool Cétostéarylique | | | | | | | | 14 % |
| Mono-stéarate de saccharose | | 26 % | 26 % | | 24 % | 24 % | | 24 % |
| di-stéarate de saccharose | 45 % | | | 45 % | | | 27 % | |
| Ricinoléate de sodium en solution | 5 % | 11 % | | 5 % | 10 % | | | 10 % |
| Cétéaryle sulfate de sodium | | | 5 % | | | | | |
| Sodium stearoyl lactylate | | | | | | 10 % | | |
| Sodium stearoyl glutamate | | | | | | | 14 % | |

On obtient ainsi dans chaque exemple une composition auto-émulsionnante, solide à température ambiante.

Les exemples suivants sont relatifs à des applications de la composition de la présente invention pour réaliser divers produits cosmétiques, tous les pourcentages étant en poids. Toutes les compositions réalisées sont stables à la centrifugation 3000 tr/min pendant 5 minutes.

### Exemple A - Préparation d'une base lavante exfoliante

On prépare cette base ayant la formulation ci-après :

| PHASE | NOM INCI | % en poids |
|---|---|---|
| A | Sodium Lauroyl Methyl Isethionate | 30,0 |
| | Cocoamido propyl betaïne | 10,0 |
| | Mélange obtenu selon un des exemples 1 à 8 | 10,0 |
| B | Water | Qs 100 |
| | Glycerin | 5,0 |
| | Acacia Senegal gum (and) xanthan gum | 1,0 |
| C | Citric acid | Qs pH=5-6 |
| | Sodium chloride | 4,5 |
| D | Fragance | 0,8 |
| | Cocos nucifera shell powder | 1,56 |
| | Sodium Benzoate | 0,5 |

Les phases A et B sont chauffées séparément jusqu'à une température de 60°C sous agitation. A est ensuite ajouté à B sous agitation modérée.

Le mélange est ensuite refroidi sous agitation douce. On ajuste la viscosité par adjonction de 4,5% de chlorure de sodium et le pH entre 5 et 6 par adjonction d'une solution d'acide citrique.

A 30°C on ajoute 0,5% de benzoate de sodium, 0,8% d'un parfum désiré et 1,56% de grains exfoliants de noix de coco.

Le produit final est une crème lavante innovante avec un aspect nacré d'origine végétale et sans ajout de produits éthoxylées généralement utilisés dans ce type d'application.

### Exemple B - Préparation d'une crème réparatrice main

On prépare comme suit cette préparation dont la formulation est ci-dessous.

On chauffe à 80°C environ la phase A.

On mélange les ingrédients de la phase B et on ajoute le mélange ainsi obtenu à la phase C préchauffée à 80°C environ.

On mélange cet ensemble pendant 10 min environ.

Puis on y ajoute la phase A précédemment préparée sous agitation modérée de type défloculeuse.

| PHASE | NOM INCI | % en poids |
|---|---|---|
| A | Propanediol dicaprylate | 5,0 |
| | Isononyl isononanoate | 3,0 |
| | Persea gratissima oil unsaponifiables | 5,0 |
| | Butyrospernum parkii (shea) butter | 15,0 |
| | Isodecyl neopentanoate | 3,0 |
| | Mélange obtenu selon un des exemples 1 à 8 | 6,0 |
| B | xanthan gum | 0,5 |
| | Glycerin | 5,0 |
| C | Water | QS 100 |
| D | Opuntia Streptacantha Stem Extract (and) Glycerin (and) Phenoxyethanol (and) Aqua | 1,0 |
| | Methylchloroisothiazolinone, Methylisothiazolinone, Magnesium Nitrate, Magnesium Chloride | 0,5 |
| | bisabosol | 2,0 |

On refroidit à 30°C le produit obtenu et on y ajoute la phase D.

On homogénéise cet ensemble pendant environ 10 min sous agitation modérée.

Le produit final se présente sous la forme d'une crème onctueuse

### Exemple C - Crème lait pour le corps

On prépare cette crème ayant la formulation ci-après :

| NOM INCI | % en poids |
|---|---|
| Propanediol dicaprylate | 25,0 |
| Mélange obtenu selon un des exemples 1 à 8 | 10,0 |
| Glycerin | 5,0 |
| Water | QS 100 |
| Benzyl alcohol (and) Dehydroacetic Acid (and) Water | 0,5 |
| Citric acid | Qs pH=5,6 |

Dans un récipient, on mélange le propanediol dicaprylate et le mélange obtenu selon un des exemples 1 à 8. On ajoute la moitié de la quantité d'eau à 80°C au mélange précédent jusqu'à l'obtention d'un gel

Puis on refroidit à 35-40°C sous agitation tout en ajoutant l'autre moitié d'eau. On ajoute alors le conservateur et on refroidit jusqu'à la température ambiante. Enfin on ajuste le pH à 5 - 6 par une solution d'acide citrique.

Cette formulation nécessite peu d'énergie mécanique et permet d'intégrer tous les ingrédients en monopote, c'est-à-dire dans une même cuve.

### Exemple D - Crème solaire

On prépare cette crème ayant la formulation ci-après :

| PHASE | NOM INCI | % en poids |
|---|---|---|
| A | Water | QS 100 |
| | Mélange obtenu selon un des exemples 1 à 8 | 10,0 |
| B | Diisopropyle sebaçate | 10,0 |
| | Octocrylene | 8,0 |
| | Benzophenone-3 | 3,0 |
| | Homosalate | 10,0 |
| | Butyl methoxydibenzoylmethane | 4,0 |
| | PVP hexadecene copolymer | 2,0 |
| | Glyceryl undecylenate | 0,5 |
| | Shorea robusta seed butter | 3,0 |
| C | Titanium dioxide (and) aluminium hydroxide (and) hydrogenated lecithin | 1,0 |
| D | Methyl propanediol | 5,0 |
| | Xanthan gum | 0,5 |
| E | Fragrance | 0,5 |

Dans un mélangeur, on introduit la phase A et on la chauffe à 80°C environ.

Dans un récipient, on chauffe la phase B à 80°C environ sous agitation : lorsque cette phase B est homogène, on y disperse la phase C.

Dans un récipient annexe, on prépare un prémix avec la phase D.

Enfin, on introduit la phase D dans le mélangeur contenant la phase A tout en maintenant la température puis la phase B.

Après refroidissement on ajoute le parfum.

### Exemple E - Déodorant

On prépare comme suit ce déodorant dont la formulation est ci-dessous.

Dans un récipient, on mélange le glyceryl undecylenate, le glyceryl caprylate et le mélange obtenu selon un des exemples 1 à 8. On ajoute la moitié de la quantité d'eau à 80°C au mélange précédent.

Puis on ajoute l'autre moitié d'eau froide. On ajoute alors la xanthan gum, l'oryza sativa (rice) hull powder et on refroidit jusqu'à la température ambiante. Enfin on ajoute l'éthanol, et le parfum puis on ajuste le pH à 5 - 6 par une solution d'acide citrique.

| NOM INCI | % en poids |
|---|---|
| Mélange obtenu selon un des exemples 1 à 8 | 8,0 |
| Glyceryl Undecylenate | 0,5 |
| Glyceryl Caprylate | 0,5 |
| Ethyl Alcohol | 10,0 |
| Water | QS 100 |
| Xanthan gum | 0,05 |
| Oryza sativa (rice) hull powder | 2,0 |
| Citric acid | Qs pH 5-6 |
| Fragrance | 0,5 |

Cette formulation permet d'intégrer tous les ingrédients en monopote.

### Exemple F - Spray

On prépare ce spray ayant la formulation ci-après :

| NOM INCI | % en poids |
|---|---|
| Mélange obtenu selon un des exemples 1 à 8 | 8,0 |
| Squalene | 5,0 |
| Glyceryl undecylenate | 0,5 |
| Water | QS 100 |
| Propanediol | 5,0 |
| Fragrance | 0,2 |
| Citric acid | QS pH 4-5 |

Dans un récipient, on mélange le glyceryl undecylenate, le squalene, le propanediol et le mélange obtenu selon un des exemples 1 à 8. On ajoute la moitié de la quantité d'eau à 80°C au mélange précédent.

Puis on ajoute l'autre moitié d'eau froide. A température ambiante on ajoute le parfum puis on ajuste le pH à 4 - 5 par une solution d'acide citrique.

Cette formulation permet d'intégrer tous les ingrédients en monopote.

### Exemple G - Lait

On prépare ce lait ayant la formulation ci-après :

| NOM INCI | % en poids |
|---|---|
| Mélange obtenu selon un des exemples 1 à 8 | 8,0 |
| Coco-caprylate/caprate | 5,0 |
| Squalene | 4,0 |
| Vegetable oil (and) hydrogenated vegetable oil (and) euphorbia cerifera (candelilla) wax | 3,0 |
| Water | QS 100 |
| Glycerin | 3,0 |
| Acacia Senegal gum (and) xanthan gum | 0,25 |
| Sodium benzoate (and) potassium sorbate | 1,0 |
| Citric acid | 0,1 |
| Fragrance | 0,3 |

Dans un récipient, on mélange tous les ingrédients excepté l'acide citrique, le conservateur et le parfum à de l'eau à 80°C. A 50°C, on ajoute le conservateur puis à 30°C le parfum. On ajuste le pH à 5 par une solution d'acide citrique.

Cette formulation permet d'intégrer tous les ingrédients en monopote.

### Exemple H - Moussant

On prépare comme suit ce produit moussant dont la formulation est ci-dessous.

Dans un récipient, on mélange tous les ingrédients excepté l'acide citrique, le conservateur et le parfum à de l'eau à 80°C. A 50°C, on ajoute le conservateur puis à 30°C le parfum. On ajuste le pH à 4,8 par une solution d'acide citrique.

| NOM INCI | % en poids |
|---|---|
| Mélange obtenu selon un des exemples 1 à 8 | 8,0 |
| Water | QS 100 |
| Sodium cocoamphoacetate ; glycerin ; lauryl glucoside ; disodium cocoyl glutamate ; sodium lauryl glucoside carboxylate | 40,0 |
| Acacia senegal gum (and) xanthan gum | 1,0 |
| Sodium benzoate | 0,5 |
| Citric acid | Qs pH=4,8 |
| Fragrance | 0,5 |

Cette formulation permet d'intégrer tous les ingrédients en monopote.

### Exemple I - Crème moussante

On prépare cette crème moussante ayant la formulation ci-après :

| PHASE | NOM INCI | % en poids |
|---|---|---|
| A | Xylitylglucoside (and) Anhydroxylitol (and) Xylitol | 3,0 |
| | Disodium EDTA | 0,1 |
| | Glycine | 0,5 |
| | Mélange obtenu selon un des exemples 1 à 8 | 5,0 |
| | Water | QSP 100 |
| B | Cocoamidopropyl Betaine | 7,2 |
| | Sodium laureth sulfate | 8,4 |
| | Sodium methyl cocoyl taurate | 3,0 |
| | Decyl glucoside | 4,0 |
| C | Glycerin | 2,5 |
| | Hydroxypropyl guar hydroxypropyltrimonium chloride | 0,3 |
| D | Sodium benzoate | 0,5 |
| | Fragrance | 0,5 |
| | Citric acid | Qs pH=4,8 |

Dans un mélangeur, on introduit la phase A et on chauffe à 80°C environ.

Dans un autre récipient, on prépare la phase B ; une fois la phase B homogène, on l'ajouter à la phase A.

Dans un récipient annexe, on prépare un prémix avec la phase C ; puis, on introduit la phase C dans le mélangeur à environ 50°C.

On refroidit à environ 30°C et on introduire la phase D ; on ajuste le pH avec une solution d'acide citrique.

### Exemple J- Crème

On prépare cette crème ayant la formulation ci-après :

| | | |
|---|---|---|
| A | Propanediol dicaprylate | 20,0 |
| | Glyceryl dibehenate (and) tribehenin (and) glyceryl behenate | 1,0 |
| | Butyrospernum parkii (shea) butter | 10,0 |
| | Glyceryl caprylate | 1,0 |
| | Glyceryl undecylenate | 1,0 |
| | Mélange obtenu selon un des exemples 1 à 8 | 6,0 |
| B | Water | Qs 100 |
| C | Propanediol | 5,0 |
| | Acacia senegal gum (and) xanthan gum | 1,0 |
| D | Ethyl linoleate (and) ethyl oleate (and) ethyl linolenate | 2,0 |
| | Opuntia streptacantha stem extract (and) glycerin (and) phenoxyethanol (and) aqua | 2,0 |

On chauffe à environ 80°C la phase A.

Dans un récipient annexe, on prépare un prémix avec la phase C : une fois obtenu ce prémix, on l'ajoute à la phase B préchauffée à 80°C environ

On mélange cet ensemble pendant 10 min environ. Puis on y ajoute la phase A, précédemment préparée, sous agitation de type défloculeuse modérée ;

On refroidit à 30°C le produit obtenu et on y ajoute la phase D.

Le produit final se présente sous la forme d'une crème onctueuse

## Revendications

1. Composition auto-émulsionnante comprenant exclusivement, par rapport au poids total des ingrédients :
- 5 à 45 % en poids d'un ester de polyglycérols,
- 5 à 60% en poids d'un ou de plusieurs esters de saccharose de HLB différente,
- 20 à 50 % en poids d'un ester de glycérol,
- 1 à 30 % en poids d'un alcool gras,
- 1 à 30 % en poids d'un agent tensioactif, **caractérisée par le fait que** ledit agent tensioactif comprend un agent choisi dans le groupe constitué par des savons d'acide gras, des savons estérifiés et des sels d'alcool gras liés par une liaison sulfate, et que tous ces ingrédients sont d'origine végétale.

2. Composition selon la revendication 1, **caractérisée par le fait que** la quantité d'ester de polyglycérols est comprise entre 5 et 35% en poids et en particulier entre 10 et 30% en poids.

3. Composition selon la revendication 2, **caractérisée par le fait que** l'ester de polyglycérols est choisi parmi les esters issus de la réaction de polyglycérols comprenant de 2 à 12 unités de glycérol, de préférence de 3 à 6 unités glycérol, avec au moins une huile végétale, partiellement hydrogénée ou non hydrogénée, d'indice d'iode compris entre 1 et 145, et en particulier de 5 à 105.

4. Composition selon la revendication 1, **caractérisée par le fait que** la quantité d'esters de saccharose est comprise entre 10 et 50% en poids et en particulier entre 20 et 45% en poids.

5. Composition selon la revendication 4, **caractérisée par le fait que** le ou les esters de saccharose sont choisis parmi les esters issus de la réaction du saccharose avec des acides gras comprenant de 10 à 22 atomes de carbone, de préférence de 12 à 20 atomes de carbone, en particulier de 12 à 18 atomes de carbone.

6. Composition selon la revendication 5, **caractérisée par le fait que** les acides gras sont choisis parmi l'acide laurique, l'acide myristique, l'acide palmitique, et l'acide stéarique ou un mélange d'au moins deux de ces acides gras.

7. Composition selon la revendication 1, **caractérisée par le fait que** l'ester de glycérol choisi parmi les esters issus de la réaction de la glycérine avec des acides gras ou de la transestérification de triglycérides d'acides gras et de glycérine, les acides gras comprenant de 8 à 22 atomes de carbone, de préférence 10 à 20 atomes de carbone, mieux de 14 à 18 atomes de carbone.

8. Composition selon la revendication 7, **caractérisée par le fait que** les acides gras sont choisis parmi l'acide myristique, l'acide palmitique, l'acide stéarique ou un mélange d'au moins deux de ces acides.

9. Composition selon la revendication 7, **caractérisée par le fait que** la quantité d'ester de glycérol peut être estérifiée en plus par un acide tel que les acides acétique, lactique, critique ou tartrique.

10. Composition selon la revendication 7, **caractérisée par le fait que** la quantité d'ester de glycérol est comprise entre 15 à 45% en poids, encore mieux de 20 à 40% en poids par rapport au poids total de la composition.

11. Composition selon la revendication 1, **caractérisée par le fait que** la quantité d'agent tensio-actif est comprise entre 1 et 20 % en poids.

12. Composition selon la revendication 11, **caractérisée par le fait que** l'agent tensio-actif est un savon d'acides gras ou savons estérifiés comprenant de 2 à 22 atomes de carbone, et préférentiellement de 3 à 20 atomes de carbone.

13. Composition selon la revendication 1, **caractérisée par le fait que** la quantité d'alcool gras est comprise entre 1 à 20% en poids, encore mieux entre 8 et 20% en poids par rapport au poids total de la composition.

14. Composition selon la revendication 13, **caractérisée par le fait que** l'alcool gras comprend de 8 à 22 atomes de carbone, les chaînes carbonées sont saturées ou insaturées ou fonctionnalisées ou linéaires ou un mélange de celles-ci, de préférence de 10 à 18 atomes de carbone, et en particulier de 12 à 18 atomes de carbone.

15. Composition selon l'une quelconque des revendications 1 à 14, **caractérisée par le fait que** les ingrédients sont d'origine végétale.

16. Application de la composition selon l'une quelconque des revendications 1 à 15 pour l'obtention de produits cosmétiques.

## Patentansprüche

1. Selbstemulgierende Zusammensetzung aufweisend ausschließlich, bezogen auf die Gesamtmenge der Zusammensetzung:
- 5 bis 45 Gew.-% Polyglycerolester,
- 5 bis 60 Gew.-% eines oder mehrerer Saccharoseester mit unterschiedlichen HLB-Werten,
- 20 bis 50 Gew.-% eines Glycerolesters,
- 1 bis 30 Gew.-% eines Tensides, **dadurch gekennzeichnet, dass** das Tensid ein Mittel, ausgewählt aus einer Gruppe bestehend aus Fettsäureseifen, veresterte Seifen und durch eine Sulfatbindung gebundene Fettalkoholsalze, umfasst und dadurch, dass alle Inhaltstoffe pflanzlichen Ursprungs sind.

2. Zusammensetzung gemäß Anspruch 1, **dadurch gekennzeichnet, dass** die Menge an Polyglycerolester zwischen 5 und 35 Gew.-%, insbesondere zwischen 10 und 30 Gew.-%, liegt.

3. Zusammensetzung gemäß Anspruch 2, **dadurch gekennzeichnet, dass** der Polyglycerolester ausgewählt ist aus Estern, die sich aus der Reaktion von Polyglycerolen aufweisend 2 bis 12 Glycerineinheiten, und vorzugsweise 3 bis 6 Glycerineinheiten, mit mindestens einem teilweise hydrierten oder unhydrierten Pflanzenöl mit einem Jodwert zwischen 1 und 145, und insbesondere zwischen 5 bis 105, ergeben.

4. Zusammensetzung gemäß Anspruch 1, **dadurch gekennzeichnet, dass** die Menge an Saccharoseester zwischen 10 und 50 Gew.-% und vorzugsweise zwischen 20 und 45 Gew.-% liegt.

5. Zusammensetzung gemäß Anspruch 4, **dadurch gekennzeichnet, dass** der oder die Saccharoseester ausgewählt sind aus Estern, die sich aus der Reaktion von Saccharose mit Fettsäuren mit 10 bis 22 Kohlenstoffatomen, vorzugsweise 12 bis 20 Kohlenstoffatomen, insbesondere 12 bis 18 Kohlenstoffatomen, ergeben.

6. Zusammensetzung gemäß Anspruch 5, **dadurch gekennzeichnet, dass** die Fettsäuren ausgewählt sind aus Laurinsäure, Myristinsäure, Palmitinsäure und Stearinsäure oder einer Mischung aus mindestens zwei dieser Fettsäuren.

7. Zusammensetzung gemäß Anspruch 1, **dadurch gekennzeichnet, dass** der Glycerolester ausgewählt ist aus Estern, die sich aus der Reaktion von Glycerin mit Fettsäuren oder Umesterung von Fettsäuretriglyceriden und Glycerin ergibt, wobei die Fettsäuren 8 bis 22 Kohlenstoffatome, vorzugsweise 10 bis 20 Kohlenstoffatome, insbesondere 14 bis 18 Kohlenstoffatome aufweisen.

8. Zusammensetzung gemäß Anspruch 7, **dadurch gekennzeichnet, dass** die Fettsäuren ausgewählt sind aus Myristinsäure, Palmitinsäure, Stearinsäure oder einer Mischung aus mindestens zwei dieser Säuren.

9. Zusammensetzung gemäß Anspruch 7, **dadurch gekennzeichnet, dass** die Menge an Glycerolester weiterhin durch eine Säure wie Essig-, Milch-, Zitronen- oder Weinsäure verestert werden kann.

10. Zusammensetzung gemäß Anspruch 7, **dadurch gekennzeichnet, dass** die Menge an Glycerolester zwischen 15 und 45 Gew.-% beträgt, bevorzugt zwischen 20 und 40 Gew.-%, bezogen auf das Gesamtgewicht der Zusammensetzung.

11. Zusammensetzung gemäß Anspruch 1, **dadurch gekennzeichnet, dass** die Menge der Tenside zwischen 1 und 20 Gew.-% liegt.

12. Zusammensetzung gemäß Anspruch 11, **dadurch gekennzeichnet, dass** das Tensid eine Seife aus Fettsäuren oder Esterseifen mit 2 bis 22 Kohlenstoffatomen, und vorzugsweise 3 bis 20 Kohlenstoffatomen, ist.

13. Zusammensetzung gemäß Anspruch 1, **dadurch gekennzeichnet, dass** die Menge an Fettalkohol zwischen 1 und 20 Gew.-%, vorzugsweise zwischen 8 und 22 Gew.-%, bezogen auf das Gesamtgewicht der Zusammensetzung, liegt.

14. Zusammensetzung gemäß Anspruch 13, **dadurch gekennzeichnet, dass** der Fettalkohol 8 bis 22 Kohlenstoffatome, vorzugsweise 10 bis 18 Kohlenstoffatome, und insbesondere 12 bis 18 Kohlenstoffatome, aufweist, wobei die Kohlenstoffketten gesättigt oder ungesättigt oder funktionalisiert oder linear oder eine Mischung davon sind.

15. Zusammensetzung gemäß einem der Ansprüche 1 bis 14, **dadurch gekennzeichnet, dass** die Inhaltsstoffe pflanzlichen Ursprungs sind.

16. Verwendung der Zusammensetzung gemäß einem der Ansprüche 1 bis 15 zur Herstellung von kosmetischen Produkten.

## Claims

1. Self-emulsifying composition comprising exclusively, relative to the total weight of the ingredients:
- 5 to 45% by weight of an ester of polyglycerol,
- 5 to 60% by weight of one or more esters of saccharose of different HLB,
- 20 to 50% by weight of an ester of glycerol,
- 1 to 30% by weight of a fatty alcohol,
- 1 to 30% by weight of a surfactant, **characterized in that** said surfactant comprises an agent selected from the group constituted by fatty acid soaps, esterified soaps and fatty alcohol salts bonded by a sulphate bond, and that all these ingredients are of plant origin.

2. Composition according to claim 1, **characterized in that** the quantity of ester of polyglycerol is between 5 and 35% by weight and in particular between 10 and 30% by weight.

3. Composition according to claim 2, **characterized in that** the ester of polyglycerol is selected from esters resulting from the reaction of polyglycerols comprising from 2 to 12 glycerol units, preferably from 3 to 6 glycerol units, with at least one vegetable oil, partially hydrogenated or not hydrogenated to an iodine value between 1 and 145, and in particular from 5 to 105.

4. Composition according to claim 1, **characterized in that** the quantity of esters of saccharose is between 10 and 50% by weight and in particular between 20 and 45% by weight.

5. Composition according to claim 4, **characterized in that** the ester or esters of saccharose are selected from the esters resulting from the reaction of saccharose with fatty acids comprising from 10 to 22 carbon atoms, preferably from 12 to 20 carbons and in particular from 12 to 18 carbon atoms.

6. Composition according to claim 5, **characterized in that** the fatty acids are selected from lauric acid, myristic acid, palmitic acid, stearic acid or a mixture of at least two of these fatty acids.

7. Composition according to claim 1, **characterized in that** the glycerol ester is selected from esters resulting from the reaction of glycerine with fatty acids or from the transesterification of triglycerides of fatty acids and glycerine, the fatty acids comprising from 8 to 22 carbon atoms, preferably from 10 to 20 carbons and more preferably from 14 to 18 carbon atoms.

8. Composition according to claim 7, **characterized in that** the fatty acids are selected from myristic acid, palmitic acid, stearic acid or a mixture of at least two of these acids.

9. Composition according to claim 7, **characterized in that** the quantity of glycerol ester may be esterified in addition by an acid such as acetic, lactic, citric or tartric acid.

10. Composition according to claim 7, **characterized in that** the quantity of glycerol ester is between 15 and 45% by weight and more preferably from 20 to 40% by weight relative to the total weight of the composition.

11. Composition according to claim 1, **characterized in that** the quantity of surfactant is between 1 and 20% by weight.

12. Composition according to claim 11, **characterized in that** the surfactant is a fatty acid soap or esterified soaps comprising from 2 to 22 carbon atoms and preferably from 3 to 20 carbon atoms.

13. Composition according to claim 1, **characterized in that** the quantity of fatty alcohol is between 1 and 20% by weight and more preferably between 8 and 20% by weight relative to the total weight of the composition.

14. Composition according to claim 13, **characterized in that** the fatty alcohol comprises from 8 to 22 carbon atoms and the carbon chains are saturated or unsaturated or functionalized or linear or a mixture thereof, preferably from 10 to 18 carbon atoms and in particular from 12 to 18 carbon atoms.

15. Composition according to any one of claims 1 to 14, **characterized in that** the ingredients are of plant origin.

16. Application of the composition according to any one of claims 1 to 15 for the production of cosmetic products.
